(19) Europäisches Patentamt
European Patent Office
Office européen des brevets

(11) Veröffentlichungsnummer: **0 187 138 B1**

(12) **EUROPÄISCHE PATENTSCHRIFT**

(45) Veröffentlichungstag der Patentschrift: **15.04.92**

(21) Anmeldenummer: **86100066.9**

(22) Anmeldetag: **03.01.86**

(51) Int. Cl.[5]: **C12N 15/55**, C12N 1/20, //C12N9/78,(C12N1/20, C12R1:19,1:40)

# (54) Mikroorganismus und Plasmid für konstitutive Creatinamidino-hydrolase-Bildung sowie Verfahren zur Herstellung derselben.

(30) Priorität: **04.01.85 DE 3500184**

(43) Veröffentlichungstag der Anmeldung:
**09.07.86 Patentblatt 86/28**

(45) Bekanntmachung des Hinweises auf die Patenterteilung:
**15.04.92 Patentblatt 92/16**

(84) Benannte Vertragsstaaten:
**AT BE CH DE FR GB IT LI LU NL SE**

(56) Entgegenhaltungen:
**DE-A- 2 659 878**

**AGRICULTURAL AND BIOLOGICAL CHEMISTRY, Band 40, Nr. 5, 1976, Seiten 1011-1018, JP; D. TSURU et al.: "Creatinine decomposing enzymes in pseudomonas putida"**

(73) Patentinhaber: **BOEHRINGER MANNHEIM GMBH**
**Patentabteilung, Abt. E Sandhofer Strasse 112-132 Postfach 31 01 20**
**W-6800 Mannheim 31 Waldhof(DE)**

(72) Erfinder: **Schumacher, Günter, Dr.**
**Kapellenweg 20**
**W-8139 Bernried(DE)**
Erfinder: **Buckel, Peter, Dr.**
**Eichenstrasse 19**
**W-8139 Bernried(DE)**
Erfinder: **Beaucamp, Klaus, Dr.**
**Von-Kühlmann-Strasse 15**
**W-8132 Tutzing(DE)**

(74) Vertreter: **Weickmann, Heinrich, Dipl.-Ing. et al**
**Patentanwälte H.Weickmann, Dr. K.Fincke F.A. Weickmann, B. Huber Dr. H. Liska, Dr. J. Prechtel Kopernikusstrasse 9 Postfach 86 08 20**
**W-8000 München 86(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

GENE, Band 16, 1981, Seiten 237-247, Elsevier Press, Amsterdam, NL; M. Bagdasarian et al.: "Specific-purpose plasmid cloning vectors. II Broad host range, high copy number, RSF1010-derived vectors, and a host-vector system for gene cloning in Pseudomonas"

**Beschreibung**

Das Enzym Creatinamidino-Hydrolase EC 3.5.3.3 findet industriell Anwendung zur Creatininbestimmung. Es wird daher u. a. in der klinischen Analytik für die Diagnose von Nierenerkrankungen verwendet, bei welchen im Serum oder im Urin Creatiningehalte auftreten, die von denen des gesunden Organismus abweichen. Zwar sind Mikroorganismen bekannt, wie beispielsweise Pseudomonas-Arten, welche unter Induktion durch Creatin in der Lage sind, Creatinamidino-Hydrolase in einer die Aufarbeitung lohnenden Menge herzustellen, die erzielbaren Ausbeuten und die Kosten der Isolierung des Enzyms stellen jedoch immer noch einen limitierenden Faktor für die industrielle Anwendung des Enzyms dar.

Es besteht daher ein Bedarf an Mikroorganismen, welche diese Nachteile nicht aufweisen und insbesondere die Creatinamidinohydrolase konstitutiv bilden, d. h. ohne daß hierzu eine Induktion erforderlich ist, und dabei wesentlich bessere Ausbeuten liefern als die bisher bekannten Creatinamidino-Hydrolasebildner. Weiter ist es ein Ziel der Erfindung, nach den Methoden der Gentechnologie einen derartigen Mikroorganismus herzustellen, bei welchem die genetische Information für eine hohe Syntheseleistung an dem angegebenen Enzym in einem Wirtsmikroorganismus vorliegt, der sich gut züchten läßt und aus dem sich das Enzym kostengünstig isolieren läßt.

Erfindungsgemäß gelingt es, diese Aufgabe zu lösen. Ein Gegenstand der Erfindung ist daher ein Mikroorganismus der Gattung E. coli oder Pseudomonas putida, welcher dadurch gekennzeichnet ist, daß er mit einem rekombinanten Plasmid transformiert ist, welches Creatinamidinohydrolase kodierende DNA enthält und konstitutiv Creatinamidinohydrolase bildet. FÜr Mikroorganismen der Gattung E. coli ist eine auch induktive Bildung dieses Enzyms bisher nicht gefunden worden. Bei Pseudomonas putida ist zwar eine Information für die Bildung von Creatinamidino-Hydrolase vorhanden, das Enzym wird jedoch nur unter Induktion gebildet und in recht niedrigen Aktivitäten.

Ein bevorzugter Mikroorganismus der Gattung E. coli gemäß der Erfindung enthält das Plasmid pBT 2a-1. Ein derartiger Mikroorganismus ist in der Lage, bis zu 50% seiner gesamten Syntheseleistung an Protein für die Bildung von Creatinamidino-Hydrolase aufzubringen.

Ein weiterer bevorzugter Mikroorganismus gemäß der Erfindung ist ein solcher der Gattung E. coli oder Pseudomonas putida, welcher das Plasmid pBT 306.16 enthält. Derartige Mikroorganismen sind ebenfalls konstitutive Creatinamidinohydrolasebildner mit sehr hoher Syntheseleistung.

Ein weiterer Gegenstand der Erfindung sind die Plasmide pBT 2a-1, DSM 3148P und pBT 306.16, DSM 3149P. Während das erstgenannte Plasmid eine besonders hohe Syntheseleistung in Mikroorganismen der Gattung E. coli liefert, besitzt das zweitgenannte Plasmid den Vorteil, daß es sowohl in der Gattung E. coli, als auch in der Gattung Pseudomonas putida eine hohe Expression des gewünschten Enzyms ergibt.

Wie bereits erwähnt, lassen sich die erfindungsgemäßen Mikroorganismen bzw. Plasmide nach Methoden der Gentechnologie gewinnen. So besteht das erfindungsgemäße Verfahren zur Herstellung von konstitutiv Creatinamidino-Hydrolase bildenden Mikroorganismen der genannten Gattungen darin, daß man DNA aus Pseudomonas putida

a) mit Eco R I limitiert verdaut und ein 5,8 Kb-Bruchstück gewinnt oder

b) mit Eco R1 und Pvu II spaltet und ein 2,2 Kb-Bruchstück gewinnt,

das nach a) oder b) erhaltene Bruchstück in einen mit dem bzw. den gleichen Restriktionsendonukleasen gespaltenen Vektor kloniert, diesen in einen für den gewählten Vektor geeigneten E. coli oder P. putida Stamm transformiert und die konstitutiv Creatinamidinohydrolase bildenden Klone isoliert. Kb steht hier für "Kilobasenpaar", also tausend Nukleotidbasenpaare.

Die Information für die Expression der Creatinamidino-Hydrolase findet sich auf dem 5,8 Kb-Bruchstück, bzw. dessen Unterfragment von 2,2 Kb, welches in der oben erwähnten Weise mit der Restriktionsendonuklease Eco R1 alleine oder Eco R1 zusammen mit Pvu II herausgespalten wird. Die jeweiligen Fragmente werden nach den dem Gentechniker bekannten Methoden in einen Vektor kloniert, der mit der gleichen bzw. den gleichen Restriktionsendonukleasen gespalten wurde, um passende Enden zu schaffen. Alternativ, wenn auch nicht bevorzugt, ist es auch möglich, für E. coli oder Pseudomonas putida geeignete Vektoren mit anderen Restriktionsendonukleasen zu spalten, deren Spaltungsstellen am speziellen Vektor so angeordnet sind, daß die Replikationsfähigkeit des an der Spaltungsstelle mit einem der oben erwähnten DNA-Bruchstücke aus Pseudomonas putida ergänzten Vektors und seine Transformierbarkeit in einen Wirtsstamm aufrechterhalten bleibt. Als Vektor verwendet man vorzugsweise das Plasmid pBR 322 und transformiert dieses nach dem Einfügen eines der beiden Pseudomonas putida DNA-Fragmente in einen geeigneten E. coli Stamm. Dem Fachmann sind zahlreiche E. coli Stämme bekannt, die sich mit dem Plasmid pBR 322 und seinen Derivaten sehr gut transformieren lassen. Ein anderer bevorzugter Vektor ist der λ-Phage Charon 10. pBR 322 sowie Derivate desselben sind ebenso wie λ-Vektoren kommerziell erhältlich.

Mehr bevorzugt wird ein Verfahren, bei dem man pBR 322 mit Eco R1 und Pvu II spaltet, das dabei gebildete 2,3 Kb-Fragment isoliert und mit dem 2,2 Kb Eco R1-Pvu II Fragment aus P. putida verknüpft unter Bildung eines als pBT 3-2 bezeichneten neuen Plasmids, welches man in E. coli transformiert. Man erhält so E. coli K12 ED 8654 DSM 3144.

In der obenbeschriebenen Weise mit dem Plasmidderivat aus pBR 322 transformierte E. coli Stämme lassen sich sehr gut aufgrund ihrer Ampicillinresistenz selektieren. Da sie sowohl ampicillinresistent als auch Creatinamidino-Hydrolasebildner sind, können nicht transformierte Zellen nicht anwachsen und unter den angewachsenen Zellen lassen sich leicht diejenigen, welche das gewünschte Enzym bilden, nach einer weiter unten näher beschriebenen Methode herausfinden.

Besonders gute Ergebnisse erhält man erfindungsgemäß, wenn man die pBT 3-2 enthaltenden transformierten E. coli Zellen zum Zeitpunkt der Amplifikation des Plasmides mit Nitrosoguanidin behandelt, danach das Plasmid daraus isoliert, erneut in E. coli Zellen transformiert, diesen Zyklus gegebenenfalls wiederholt und aus den dabei erhaltenen Mikroorganismusklonen mit besonders ausgeprägter Creatinamidino-Hydrolase-Aktivität das Plasmid pBT 2a-1, DSM 3148P, welches ebenfalls einen Gegenstand der Erfindung darstellt, gewinnt. Wie oben bereits erwähnt, produzieren E. coli Zellen, welche dieses Plasmid enthalten, bis zu 50% an Creatinamidino-Hydrolase, bezogen auf ihre gesamte Proteinbildung.

Ein Screeningsystem, welches es gestattet, gebildete Mikroorganismenklone mit besonders hoher Aktivität an Creatinamidino-Hydrolase zu identifizieren, erhält man, indem man derartige Klone mit Agaroseplatten kontaktiert, welche Creatin, Sarcosinoxidase, Peroxidase und ein $H_2O_2$-Farbindikatorsystem gelöst enthalten. Man wählt dann diejenigen Klone für die Vermehrung aus, welche die stärkste Färbung entsprechend der stärksten Enzymbildung ergeben. Als $H_2O_2$-Farbindikatorsystem wird bevorzugt 4-Aminoantipyrin in Kombination mit N-Ethyl-N-(sulfoethyl)-3-methylanilinsalz, zweckmäßig das Kaliumsalz, verwendet.

Mit dem erfindungsgemäßen Verfahren lassen sich auch Plasmide herstellen, die nicht nur zur Expression des Enzyms in E. coli, sondern auch in P. putida geeignet sind. Vorzugsweise verfährt man hierfür so, daß man aus pBT 2a-1 durch Spaltung mit den Restriktionsendonukleasen Pvu I und Pvu II ein 2,8 Kb-Fragment gewinnt und dieses mit einem weiteren, 10 Kb-Fragment ligiert, welches aus pBT 306.1 durch Spaltung mit Pvu I und Sma I erhalten wird. Man erhält so das Plasmid pBT 306.16, DSM 3149P, welches die konstitutive Creatinamidino-Hydrolase-Bildung sowohl in E. coli als auch in P. putida bewirkt.

Es ist überraschend, daß erfindungsgemäß eine wesentliche Erhöhung der Enzymbildung erreicht wird. Es wurde nämlich zwar schon mehrfach berichtet, daß durch die Anwendung der Methoden der DNA-Neukombination durch Erhöhung der Kopienzahl eines bestimmten Gens eine gesteigerte Genexpression gelungen ist. Es kann jedoch nicht davon ausgegangen werden, daß die Übertragung von Genen aus Pseudomonas in E. coli mit Wahrscheinlichkeit zu einer Erhöhung der Genexpression führt. Tatsächlich ist sogar für die Mehrzahl der durch DNA-Neukombination aus Pseudomonas in E. coli übertragene Gene eine Reduktion der Genexpression berichtet worden (vgl. z. B. Stanisisch und Ortiz, J. Gen. Microbiol., 1976, 94, 281-289, Nakazawa et al., J. Bacteriol., 1978, 134, 270-277, Ribbons et al., Soc. Gen. Microbiol., Quart., 1978, 6, 24-25, Franklin et al., in Microbiol Degradation of Xenobiotics and Recalcitrant Compounds, Leisinger Cook, Hütter und Nuesch Hrsgb., 1981, 109-130). Daß eine derartige Verbesserung nicht erwartet werden kann, zeigt eine Betrachtung der verschiedenen biologischen Syntheseschritte, die ablaufen müssen, bis endlich ein enzymatisch aktives Protein gebildet wird.

Die Information für ein Protein ist in der Desoxyribonucleinsäure (DNA) enthalten. Diese DNA wird durch eine DNA-abhängige RNA-Polymerase in mRNA (Boten ribonucleinsäure) übersetzt. Die so synthetisierte mRNA wird an den Ribosomen in Protein übersetzt, dabei bestimmen jeweils drei Nucleotide (Triplett oder Codon) - nach den Gesetzen des genetischen Codes - den Einbau einer bestimmten Aminosäure.

Kontrollbereiche auf DNA-Ebene bestimmen, an welcher Stelle ein Strang der DNA in mRNA übersetzt wird (Promotorsequenzen) bzw. an welcher Stelle die Synthese der mRNA gestoppt wird. (Terminationssequenz).

Stopp- und Startsequenzen sind ebenso auf der Ebene der Proteinsynthese (Translation) bekannt. Dabei bestimmt im allgemeinen ein ATG (das in f-Methionin übersetzt wird) den Beginn eines Proteins und z. B. ein TAA oder ein TAG das Ende der Translation.

Das Ausmaß der Expression einer Polypeptidsequenz ist von mehreren Faktoren abhängig: z. B. u. a. von der Qualität der Promotorsequenz, mRNA-Stabilität, Sekundär- und Tertiärstruktur der mRNA, Qualität der ribosomalen Bindungsstelle, Abstand der ribosomalen Bindungsstelle vom Startcodon (ATG), Nucleotidsequenz zwischen ribosomaler Bindungsstelle und Startcodon (ATG) und dem Vorhandensein effizienter Stoppsignale auf Transskriptions- und Translationsebene. Ohne genaue Kenntnis der Primärstruktur des Gens und des von diesem kodierten Proteins kann nicht gezielt in die oben beschriebenen Regulationsprozesse der Genexpression eingegriffen werden. Da diese genauen Kenntnisse im vorliegenden Falle nicht

vorhanden waren, konnte die verbesserte Syntheseleistung der erfindungsgemäßen Mikroorganismen und Plasmide nicht vorhergesehen werden.

Im Rahmen der vorliegenden Erfindung werden als E. coli vorteilhaft Derivate des E. coli K12-Stammes verwendet. Unter diesen wurden beispielsweise erfolgreich eingesetzt:

E. coli K12, W 3350 (thi, galK, galT, rpsl, von P. Tiollais), DSM 3141
E. coli K12, ED 8654 (trp R, hsd $M^+$, hsd $R^-$, sup E, sup F, von K. Murray), DSM 2102
E. coli K12, CSH 1 (thi, trp, lac z, rpsl aus Cold Spring Harbor Stammsammlung), DSM 3142

Als Wirtszellen vom Pseudomonas putida Stamm können Wildtypisolate aber auch Laborstämme eingesetzt werden. Besonders gute Ergebnisse wurden erzielt mit Pseudomonas putida 2440, DSM 2106 (Gene 1981, 237-247).

Als Vektorsysteme für die Expression in E. coli werden erfindungsgemäß, wie erwähnt, vorzugsweise genetisch manipulierte Derivate des handelsüblichen Plasmids pBR 322 (Gene 1977, 95-113) verwendet. Für die Expression in Pseudomonas-Stämmen werden vorzugsweise genetisch abgewandelte Derivate des Plasmids pRSF 1010 (Gene 1981, 237-247) verwendet. Bei Verwendung der oben angegebenen Restriktionsendonukleasen für die Konstruktion der genetisch abgewandelten Plasmide der Erfindung erhält man die Creatinamidinohydrolase kodierende Genabschnitte, welche noch das Expressions-Vektor-System und einen Regulationsursprung, der eine erhöhte Kopienzahl des Vektorsystems in der Wirtszelle bewirkt, sowie Gene, auf deren Produkte leicht selektioniert werden kann (z. B. Antibiotika-Resistenzen) enthalten.

Im Nachstehenden wird die Erfindung in Verbindung mit der Zeichnung und den Beispielen näher beschrieben. In der Zeichnung stellen dar:

Fig. 1 eine schematische Darstellung der Herstellung des Plasmids pBT 3-2 unter Verwendung des 2,2 Kb-Fragments aus der Pseudomonas putida DNA und des Plasmids pBR 322 als Ausgangsmaterial.

Fig. 2 zeigt die erfindungsgemäße Herstellung des Plasmids pBT 306.1 aus den Plasmiden RSF 1010 und pACYC 177 und

Fig. 3 zeigt die Bildung des erfindungsgemäßen Plasmids pBT 306.16 aus pBT 306.1 und pBT 2a-1 schematisch.

Fig. 4 zeigt ein SDS Gel, in dem Zellextrakte aufgetragen sind, von Spalte 1: Ausgangsstamm Pseudomonas putida, Spalte 2: dem das Plasmid pBT 2a-1 tragenden E. coli Wirtsstamm ED, Spalte 4: Wirtsstamm ED und Spalte 3: zum Vergleich 15 μg der gereinigten Creatinase.

Fig. 5 zeigt die das Enzym Creatinamidinohydrolase kodierende DNA-Sequenz und die aus der DNA-Sequenz resultierende Proteinsequenz.

Um positive Klone, d. h. Mikroorganismenklone, welche konstitutiv Creatinamidinohydrolase bilden, herauszufinden, kann erfindungsgemäß ein Screening-System eingesetzt werden, welches nach dem Prinzip des Enzymimmunotests arbeitet. Hierbei werden spezifische Antikörper gegen die Creatinamidino-Hydrolase an einen geeigneten Träger, z. B. eine Polyvinyl-Folie fixiert, die Folie wird auf lysierte Kolonien oder auch auf Plaques aufgelegt. Nach Waschen mit $H_2O$ wird die Folie mit dem gleichen spezifischen Antikörper in Form eines Enzymkonjugats (z. B. mit Peroxidase) inkubiert. Liegt ein das Enzym produzierender Klon vor, so entsteht ein Sandwich aus Antikörper-Antigen und enzymmarkiertem Antikörper.

Das Antikörper-Peroxidase- Konjugat gibt in einem geeigneten Farbindikatorsystem eine Färbung, z. B. bei einem Indikatorsystem aus Tetramethylbenzidin, Dioctylnatriumsulfosuccinat und $H_2O_2$ in Gelatine grüne Farbflecken. Dieses System ergibt eine Nachweisgrenze von 10 pg bis 100 pg Proteinantigen. Die Herstellung eines derartigen Enzymimmunotests und die Präparation geeigneter Antikörper kann nach der Anleitung zu "Testkombination Genexpression" Boehringer Mannheim GmbH, erfolgen.

Die folgenden Beispiele erläutern die Erfindung weiter.

**Beispiel 1**

A) Isolierung der Chromosomalen Pseudomonas putida DNA

Die chromosomale DNA von Pseudomonas putida DSM 2106 wird nach Lyse der Zellen und Aufwickeln der DNA auf einen Glasstab isoliert und nach 2 Phenolisierungen und Ethanolfällung in einer Konzentration von 600 μg/ml gelöst (Cosloy und Oishi, Molec. Gen. Genet., 1973, 124, 1-10).

10 μg chromosomale DNA werden limitiert mit 5 Einheiten Eco RI, E.C. 3.1.23.11, 30 Minuten gespalten und das Ausmaß der Verdauung im Agarose Gel analysiert.

B) Isolierung und Reinigung von λ Charon 10 DNA

$10^{10}$ Bakterien des Stammes E. coli ED DSM 2102 werden mit $5x10^8$ λ Phagen Charon 10 für 20 Minuten bei 37 °C inkubiert und anschließend bis zum Beginn der Lyse der Bakterien in 500 ml Vollmedium wachsen gelassen. Die Prozedur der Phagen- und DNA-Isolierung erfolgte exakt nach der

Vorschrift von Maniatis et al., in: Molecular Cloning, Cold Spring Harbor Laboratory, 1982, 76-85.

10 µg Charon 10 DNA werden vollständig mit Eco RI gespalten. 1 µg limitiert mit Eco RI verdaute chromosomale Pseudomonas putida DNA gemäß A) wird mit 3 µg mit Eco RI gespaltener Charon 10 DNA mit 40 Einheiten des Enzyms T4 DNA Ligase inkubiert. Das Verpacken der zusammenligierten DNA-Fragmente in Kopf- und Schwanzproteine des Phagen λ erfolgt im Reagenzglas. Die Herstellung der zur Verpackung notwendigen Proteine sowie die Verpackung der DNA erfolgt nach Maniatis et al., Molecular Cloning, Cold Spring Harbor Laboratory 1982, 256-291 (in vitro Verpackungssysteme für λ DNA Partikel sind kommerziell erhältlich, z. B. Boehringer Mannheim, "DNA-Packaging Kit"). Ca. 0,5 µg der zusammengeknüpften λ und Pseudomonas putida DNA wurden mit 20 µl des in vitro Verpackungsansatzes inkubiert, nach 60 Minuten werden 0,5 ml SM Puffer (Maniatis et al., Cold Spring Harbor Laboratory 1982, 443) zugegeben und 1/200 Volumen (2,5 µl) des Verpakkungsansatzes mit 200 µl einer Übernachtkultur des Stammes ED (in $10^{-2}$ M Magnesiumsulfat) für 10 Minuten bei 37° C inkubiert. Die Bakteriensuspension wird anschließend mit 3 ml LB (Miller in Experiments in Molecular Genetics, Cold Spring Harbor Laboratory 1972 433) Agarose (0,8%) gemischt und auf LB Platten gegossen. Pro 1 µg eingesetzter DNA werden ca. $10^5$ Phagenlöcher (Plaques) erhalten.

Zur Identifizierung von Phagen, die ein Creatinase kodierendes Gen enthalten, wird der vorher beschriebene Enzym-Immunotest verwendet. Das Indikatorsystem besteht aus 6 mg/ml Tetramethylbenzidin, 20 mg/ml Dioctylnatriumsulfosuccinat und 0,01 % $H_2O_2$ in 6%iger Gelatine. Pro 1000 Plaques werden zwei positive Signale festgestellt.

C) Reklonierung des Creatinase-Gens in E. coli

Von fünf im Enzymimmunotest positiven Plaques wurde, wie vorher beschrieben, Phagen-DNA präpariert. Spaltung der fünf verschiedenen DNAs mit Eco RI zeigten neben unterschiedlichen Banden eine gemeinsame DNA-Bande in allen fünf Phagen DNAs von 5,8 Kb. Das 5,8 Kb große Fragment wurde mit verschiedenen Restriktionsnukleasen charakterisiert (Fig. 1).

Aus ca. 5 µg dieses Eco RI Fragments wurde unter Verwendung von Pvu II ein 2,2 Kb Fragment gespalten. Die entstehenden DNA Fragmente werden in einem niedrig schmelzenden Agarosegel nach ihrer Größe getrennt und das 2,2 Kb Eco RI - Pvu II Fragment isoliert. Die Isolierung von DNA-Fragmenten aus niedrigschmelzenden Agarosegelen erfolgt, indem die entsprechende Bande ausgeschnitten, in ein Reagenzglas (Eppendorfröhrchen) überführt und mit etwa dem zweifachen Volumen Wasser versetzt wird. Anschließend wird so lange bei 65° C inkubiert (5 bis 10 Minuten) bis die Agarose geschmolzen ist, die Probe wird kurz geschüttelt und mit einem halben Volumen Phenol (neutralisiert mit 10 mM TRIS-HCl pH 7,5 und 1 mM EDTA, TE) kräftig geschüttelt. Die Phasen werden durch Zentrifugation für 10 Minuten bei 15 000 g getrennt und die obere wäßrige Phase erneut mit Phenol ausgeschüttelt. Nach einer Zentrifugation von 10 Minuten bei 15 000 g wird die obere Phase zweimal mit je 1 ml Ether ausgeschüttelt, der Ether bei 65° C abgedampft und die DNA mit 1/10 Volumen 3 M Naacetat pH 7,2 und 2,5fachen Volumen Ethanol bei -20° C gefällt. Die DNA wird durch Zentrifugation für 10 Minuten bei 15 000 g sedimentiert im Vakuum getrocknet und in 10 µl TE aufgenommen. Alle weiter beschriebenen Fragmentisolierungen erfolgen nach dieser Prozedur.

Ca. 4 µg pBR 322 DNA werden mit Eco RI und Pvu II gespalten und ein 2,3 Kb Fragment isoliert. 0,2 µg dieses pBR 322 Fragments werden über Nacht unter Verwendung von fünf Einheiten T4 DNA Ligase mit 0,5 µg des 2.2 Kb Eco RI-PvuII Fragments aus dem vorher beschriebenen λ Phagen inkubiert. Das resultierende Plasmid trägt die Bezeichnung pBT 3-2 und kodiert in E. coli eine biologisch aktive Creatinase.

**Beispiel 2**

Die Creatinase codierende DNA aus Plasmid pBT 3-2 wird exakt nach der Methode von Talmadge und Gilbert, Gene, 1980, 12, 235-241, während der Amplifikationsphase mit Nitrosoguanidin behandelt. Anschließend wird die Plasmid-DNA nach Lyse der Zellen über die CsCl-Ethidiumbromid Methode isoliert (Maniatis et al., Molecular Cloning, Cold Spring Harbor 1982, 88-94). Kompetente Zellen des Stammes E. coli ED 8654 werden mit Plasmid DNA transformiert (Maniatis et al., Molecular Cloning Cold Spring Harbor, 1982, 250-251) und auf Vollmediumplatten (LB) die 20 µg/ml Ampicillin enthalten, ausplattiert. Nach Inkubation über Nacht bei 37° C werden die Kolonien auf LB Platten gestempelt, auf die zuvor ein Nitrocellulose Filterpapier (Schleicher, Schüll BA 85) aufgelegt wurde. Nach Bebrütung der Platten für 12 bis 18 Stunden bei 37° C wird der Nitrocellulosefilter mit den Kolonien abgehoben und in eine Glaspetrischale (Ø 20 cm) überführt, in die 1 ml Chloroform/Toluol (1:1) gegeben wurde. Inkubation erfolgt für 20 Minuten bei 37° C. Der Nitrocellulosefilter wird anschließend auf eine Indikator-Agaroseplatte so aufgelegt, daß ein direkter Kontakt zwischen Zellen und Indikatorplatte entsteht. Die Farbreaktion erfolgt in Abhängigkeit von der Zeit

und der Menge der in den einzelnen Klonen synthetisierten Creatinase. Aus dem oben beschriebenen Aktivitätsscreening wird der Klon ED mit dem Plasmid pBT 2a-1, DSM 3143 isoliert. Dieses Plasmid kodiert eine Creatinase, die ca. 50% des löslichen Proteins der Zellen ausmacht. Dieses Verfahren zeigt Fig. 1 schematisch.

Alternativ zu der hier beschriebenen direkten NG-Mutagenese kann auch durch Einfügung eines Fremdpromotors, z. B. des Lactosepromotors (dieser kann z. B. aus kommerziell erhältlichen Plasmiden, wie z. B. den pUC-Plasmiden, als DNA-Fragment isoliert werden), eine Expressionssteigerung der Creatinase erhalten werden. Dazu wird Plasmid pBT 3-2 an der EcoR1-Stelle geöffnet, mit der Exonuclease Bal 31 so behandelt, daß ca. 10 bis 100 Bp von jeder Seite entfernt werden. Dann wird der Lactose-Promotor mit Hilfe des Enzyms $T_4$-Ligase, unter Verknüpfung der Enden, in das verkürzte Plasmid pBT 3-2 einligiert. Diese DNA wird dann, wie oben beschrieben, mit Nitroso-Guanidin mutagenisiert, anschließend für die Transformation des Stammes ED verwendet und die Klone werden in dem beschriebenen Plattenscreening auf hohe Genexpression getestet.

Die vorstehend erwähnte Indikator-Agaroseplatte stellt ein Testsystem für ein Aktivitätsscreening dar, dessen Prinzip darin besteht, aus Creatin durch die Enzyme Creatinamidino-Hydrolase und Sarcosinoxidase das gebildete $H_2O_2$ über Peroxidase (POD) in 1/2 $O_2$ und $H_2O$ zu spalten und den Sauerstoff mit dem Farbindikatorsystem z. B. aus 4-Aminoantipyrin (4-AAP) und N-Ethyl-N-(sulfoethyl)-3-methylanilin, K-Salz (EST) reagieren zu lassen. Es entsteht eine blau-violette Färbung, die bei Überschuß der Enzyme Sarcosinoxidase und Peroxidase ein Maß für in den Kolonien synthetisierte Creatinamidino-Hydrolase darstellt.

Testprinzip:

$$\text{Creatin} + H_2O \xrightarrow{\text{Creatinamidino-Hydrolase}} \text{Sarcosin} + \text{Harnstoff}$$

$$\text{Sarcosin} + O_2 + H_2O \xrightarrow{\text{Sarc - OD}} \text{Glycin} + \text{Formaldehyd} + H_2O_2$$

$$H_2O_2 + 4\text{ - AAP} + \text{EST} \xrightarrow{\text{POD}} \text{Farbstoff} + 2\ H_2O$$

Zusammensetzung des Creatinamidino-Hydrolase Aktivitätsscreening Systems

| | | | |
|---|---|---|---|
| 1. | Creatin | 10 mM | Endkonzentration |
| 2. | Na $N_3$ | 0,5 mM | " |
| 3. | Tris HCl pH 7,8 | 20 mM | " |
| 4. | Sarcosin Oxidase | 5 U/ml | " |
| 5. | Peroxidase | 2,5 U/ml | " |
| 6. | 4-AAP | 0,25 mg/ml | " |
| 7. | EST | 1,5 mg/ml | " |

Die unter 1 bis 7 angegebenen Reagenzien werden gelöst und mit gleichem Volumen niedrig schmelzender Agarose (2%) gemischt. 6 ml werden in eine Petrischale gegossen, die Platten können ca. 2 Wochen bei 4 °C in der Dunkelheit gelagert werden.

**Beispiel 3**

Zur Klonierung und Expression der klonierten Creatinamidino- Hydrolase im Pseudomonas putida wird Plasmid RSF 1010 (Bagdasarian et al., Gene 1981, 16, 237-247) verwendet. RSF 1010 wurde mit Pvu II linearisiert und aus Plasmid pACYC 177 (Chang und Cohen, J. Bacteriol., 1978 134, 1141-1156) nach Hae II Spaltung das 1,4 Kb Fragment isoliert. 0,2 µg RSF 1010 DNA wurden mit 1 µg des Hae II Fragments unter Verwendung von T4 Ligase verknüpft, das resultierende Plasmid ist pBT 306.1 (Fig. 2). RSF 1010 und Derivate dieses Plasmids zeichnen sich durch einen weiten Wirtsbereich aus (Gene, 16 (1981) 237-247) und sind z. B. geeignet, sich sowohl in Pseudomonaden als auch in E. coli zu amplifizieren. Plasmid pBT 2a-1 wurde mit Pvu I und Pvu II gespalten und das 2,8 Kb Fragment isoliert, pBT 306.1 wird mit Pvu I und Sma I gespalten und das 10 Kb Fragment isoliert. 0,5 µg der Vektor DNA wird mit 0,5 µg des PvuI-PvuII Fragments ligiert. E. coli ED wird transformiert und Creatinase kodierende Klone werden mit Hilfe des vorher beschriebenen Platten-Aktivitätsscreenings identifiziert. Von einem der positiven Klone wird nach der vorher beschriebenen CsCl - Ethidiumbromid Methode Plasmid DNA präpariert. Das Plasmid trägt die Bezeichnung pBT 306.16, DSM 3149 P (Fig. 3).

Die Transformation von Plasmid DNA in Pseudomonas putida 2440 erfolgt exakt nach der Methode von Franklin et al. in: Microbiol Degradation of Xenobiotics and Recalcitrant Compounds, Leisinger, Cook, Hütter und Nuesch, Hrgsb., 1981, 109-130. Mit Hilfe des Plattenaktivitätsscreenings wurden positive Klone identifiziert. Dies ist bei Pseudomonas putida 2440 - obwohl dieser Stamm eine chromosomal kodierte Creatinamidino-Hydrolase enthält - möglich, da die Expression der Plasmid-kodierten Creatinamidino-Hydrolase konstitutiv erfolgt. Dieses Unterscheidungsmerkmal erlaubt es, zwischen chromosomal-kodierter und plasmid-kodierter Creatinamidino-Hydrolase zu diskriminieren.

**Beispiel 4**

Die Bestimmung der Creatinamidino-Hydrolase-Aktivität erfolgt über den Nachweis der in der Reaktionsfolge mit Urease gebildeten Ammoniumionen mit der Testkombination "Harnstoff" (Boehringer Mannheim, Best. Nr. 124770).

Der Wildtyp Pseudomonas putida 2440 wird zur Bestimmung der Creatinamidino-Hydrolase-Aktivität in LB Medium (5 ml), das 1 % Creatin enthält, bei 30 °C über Nacht inkubiert. Die Zellen werden durch Zentrifugation geerntet und einmal in 50 mM Phosphatpuffer pH 7,5 gewaschen. Die Zellen werden im ursprünglichen Volumen in Phosphatpuffer (50 mM pH 7,5) aufgenommen und durch Ultraschallbehandlung (4 x 30 Sekunden) aufgeschlossen. Anzucht und Aufschluß von Zellen, die ein Creatinamidino-Hydrolase kodierendes Plasmid enthalten, erfolgt in gleicher Weise, wie oben beschrieben, mit der Ausnahme, daß das Medium kein Creatin zur Induktion enthält und daß auf das Plasmid durch Zugabe von Ampicillin (20 µg/ml für Plasmid pBT 3-2, pBT 2a-1) bzw. Streptomycin (200 µg/ml für Plasmid pBT 306.16) selektioniert wird. Das Wachstum der Kulturen erfolgt für Pseudomonas putida bei 30 °C, für E. coli bei 37 °C.

Creatinamidino-Hydrolase in Pseudomonas putida und E. coli

| | Stamm/Plasmid | Aktivität U/l | Anzucht |
|---|---|---|---|
| 1) | Pseudomonas putida 2440 | 1 | - Creatin |
| 2) | " " " | 250 | + Creatin |
| 3) | Pseudomonas putida 2440/ pBT 306.16 | 1800 | - Creatin |
| 4) | E. coli ED | - | ± Creatin |
| 5) | E. coli ED/pBT 3-2 | 30 | - Creatin |
| 6) | E. coli ED/pBT 2a-1 | 2800 | - Creatin |

Die Daten zeigen, daß durch die Klonierung der Creatinamidino-Hydrolase 1). E. coli Bakterien die neue Eigenschaft erhalten Creatinamidino-Hydrolase zu synthetisieren und 2). diese Expression - im Gegensatz zum Ausgangsstamm Pseudomonas putida - sowohl für E. coli wie für Pseudomonas putida konstitutiv erfolgt. Des weiteren ist zu entnehmen, daß durch Mutagenese der Creatinamidino-Hydrolase kodierenden DNA eine besonders hohe Expression erzielt werden kann. (Erhöhung der Liter-Aktivität gegenüber dem nicht induzierten Ausgangsstamm, bei Pseudomonas Faktor 1800, bei E. coli Faktor 2800).

In E. coli ED/pBT 2a-1 DSM 3143 beträgt die Aktivität 500 Einheiten/g Biomasse (feucht) bzw. die spezifische Aktivität 4,5 U/mg Protein. Da die spez. Aktivität des hochgereinigten Proteins 9 U/mg beträgt, heißt das, daß die Creatinamidino-Hydrolase in E. coli 50 % des löslichen Proteins ausmacht. Analyse eines Rohextrakts im SDS-Gel (Laemmli, Nature, 1970, 227, 680-685) zeigt, daß die Creatinamidino-Hydrolase die Hauptbande der löslichen Proteinfraktion darstellt (Fig. 4, Spalte 2).

**Beispiel 5**

Für die Kultivierung im Fermenter wurden drei verschiedene E. coli-Wirtsysteme, nämlich E. coli W 3350, E. coli ED 8654 bzw. E. coli CSH 1 verwendet. Das Plasmid pBT 2a-1 wird in die entsprechenden kompetenten Zellen transformiert. Nach Reinigung auf Einzelkolonien wird eine Vorkultur in DYT Medium (Miller, Experiments in Molecular Genetics, Cold Spring Harbor, 1972, 433) das 20 µg/ml Ampicillin enthält, über Nacht bei 37 °C angezogen. Das Fermentationsmedium (DYT) wird mit der Vorkultur beimpft (Inocculum 1 %) und ohne Selektion auf Plasmiderhalt 20 bis 30 Stunden bei 37 °C wachsen lassen. Die Creatinamidino-Hydrolase-Aktivität beträgt nach 25 Stunden ca. 600 U/g Feuchtmasse bzw. 4,5 U/mg Protein.

Für die Fermentation von Pseudomonas putida wird das Plasmid pBT 306.16 in kompetente Zellen des Stammes 2440 wie vorher beschrieben, transformiert, wobei man PS putida DSM 3147 erhält.

Nach Reinigung von Einzelkolonien wird eine Vorkultur in DYT Medium, das 200 µg/ml Streptomycin enthält, bei 30 °C über Nacht inkubiert. Das Fermentationsmedium (DYT) wird beimpft (Inocculum 1%) und die Kultur 20 bis 30 Stunden bei 30 °C wachsen gelassen. Die Aktivität nach 25 Stunden beträgt ca. 220 U/g Feuchtmasse bzw. 1,8 U/mg Protein.

**Patentansprüche**
**Patentansprüche für folgende Vertragsstaaten : BE, CH, DE, FR, GB, IT, LI, LU, NL, SE**

1. Mikroorganismus der Gattung E.coli oder Pseudomonas putida,
**dadurch gekennzeichnet,**
daß er konstitutiv Creatinamidinohydrolase bildet und erhältlich ist durch Transformation mit einem rekombinanten Plasmid, welches Creatinamidinohydrolase codierende DNA enthält, welche gewonnen wurde aus Pseudomonas putida-DNA
   a) durch limitierte Verdauung mit Eco R I und Isolierung eines 5,8 kb Bruchstücks oder
   b) durch Spaltung mit Eco R I und Pvu II und Isolierung eines 2,2 kb Bruchstücks.

2. Mikroorganismus der Gattung E. coli gemäß Anspruch 1, **dadurch gekennzeichnet,** daß er das Plasmid pBT 2a-1, DSM 3148P enthält.

3. Mikroorganismus der Gattung E. coli oder Pseudomonas putida gemäß Anspruch 1, **dadurch gekennzeichnet,** daß er das Plasmid pBT 306.16, DSM 3149P enthält.

4. Rekombinierte DNA, **dadurch gekennzeichnet,** daß sie die für das Creatin spaltende Protein der angegebenen Aminosäuresequenz 1 bis 403 codierende Basensequenz 1 bis 1212

MetGlnMetProLysThrLeuArgIleArgAsnGlyAspLysValArgSerThrPheSer
ATGCAAATGCCCAAGACGCTCCGCATCCGTAACGGCGACAAGGTTCGCTCCACCTTCTCC

AlaGlnGluTyrAlaAsnArgGlnAlaArgLeuArgAlaHisLeuAlaAlaGluAsnIle
GCCCAGGAATACGCCAATCGCCAAGCCAGGCTGCGCGCCCACCTGGCGGCGGAGAACATC

AspAlaAlaIlePheZhrSerTyrHisAsnIleAsnTyrTyrSerAspPheLeuTyrCys
GACGCCGCGATCTTCACCTCGTACCACAACATCAACTACTACTCCGACTTCCTCTACTGC

SerPheGlyArgProTyrAlaLeuValValThrHisAspAspValIleSerIleSerAla
TCCTTCGGCCGCCCCTACGCGTTGGTGGTGACCCACGACGACGTCATCAGCATCAGCGCC

AsnIleAspGlyGlyGlnProTrpArgArgThrValGlyThrAspAsnIleValTyrThr
AACATCGACGGCGGCCAGCCGTGGCGCCGCACCGTCGGCACCGACAACATCGTCTACACC

AspTrpGlnArgAspAsnTyrPheAlaAlaIleGlnGlnAlaLeuProLysAlaArgArg
GACTGGCAGCGCGATAACTACTTCGCCGCCATCCAGCAGGCGTTGCCGAAGGCCCGCCGC

IleGlyIleGluHisAspHisLeuAsnLeuGlnAsnArgAspLysLeuAlaAlaArgTyr
ATCGGCATCGAACATGACCACCTGAACCTGCAGAACCGCGACAAGCTGGCCGCGCGCTAT

ProAspAlaGluLeuValAspValAlaAlaAlaCysMetArgMetArgMetIleLysSer
CCGGACGCCGAGCTGGTGGACGTGGCCGCCGCCTGCATGCGTATGCGCATGATCAAATCC

AlaGluGluHisValMetIleArgHisGlyAlaArgIleAlaAspIleGlyGlyAlaAla
GCCGAAGAGCACGTGATGATCCGCCACGGCGCGCGCATCGCCGACATCGGTGGTGCGGCG

ValValGluAlaLeuGlyAspGlnValProGluTyrGluValAlaLeuHisAlaThrGln
GTGGTCGAAGCCCTGGGCGACCAGGTACCGGAATACGAAGTGGCGCTGCATGCCACCCAG

AlaMetValArgAlaIleAlaAspThrPheGluAspValGluLeuMetAspThrTrpThr
GCCATGGTCCGCGCCATTGCCGATACCTTCGAGGACGTGGAGCTGATGGATACCTGGACC

TrpPheGlnSerGlyIleAsnThrAspGlyAlaHisAsnProValThrThrArgLysVal
TGGTTCCAGTCCGGCATCAACACCGACGGCGCGCACAACCCGGTGACCACCCGCAAGGTG

AsnLysGlyAspIleLeuSerLeuAsnCysPheProMetIleAlaGlyTyrTyrThrAla
AACAAGGGCGACATCCTCAGCCTCAACTGCTTCCCGATGATCGCCGGCTACTACACCGCG

LeuGluArgThrLeuPheLeuAspHisCysSerAspAspHisLeuArgLeuTrpGlnVal
TTGGAGCGCACCCTGTTCCTCGACCACTGCTCGGACGACCACCTGCGTCTGTGGCAGGTC

AsnValGluValHisGluAlaGlyLeuLysLeuIleLysProGlyAlaArgCysSerAsp
AACGTCGAGGTGCATGAAGCCGGCCTGAAGCTGATCAAGCCCGGTGCGCGTTGCAGCGAT

IleAlaArgGluLeuAsnGluIlePheLeuLysHisAspValLeuGlnTyrArgThrPhe
ATCGCCCGCGAGCTGAACGAGATCTTCCTCAAGCACGACGTGCTGCAGTACCGCACCTTC

GlyTyrGlyHisSerPheGlyThrLeuSerHisTyrTyrGlyArgGluAlaGlyLeuGlu
GGCTACGGCCACTCCTTCGGCACGCTCAGCCACTACTACGGCCGCGAGGCCGGGTTGGAA

LeuArgGluAspIleAspThrValLeuGluProGlyMetValValSerMetGluProMet
CTGCGCGAGGACATCGACACCGTGCTGGAGCCGGGCATGGTGGTGTCGATGGAGCCGATG

IleMetLeuProGluGlyLeuProGlyAlaGlyGlyTyrArgGluHisAspIleLeuIle
ATCATGCTGCCGGAAGGCCTGCCGGGCGCCGGTGGCTATCGCGAGCACGACATCCTGATC

ValAsnGluAsnGlyAlaGluAsnIleThrLysPheProTyrGlyProGluLysAsnIle
GTCAACGAGAACGGTGCCGAGAACATCACCAAGTTCCCCTACGGCCCGGAGAAAAACATC

IleArgLys
ATCCGCAAATGA

oder ein nach dem genetischen Code für die gleiche Aminosäurensequenz codierendes Äquivalent davon enthält.

**5.** Rekombinierte DNA nach Anspruch 4 in Form des Plasmids pBT 2a-1, DSM 3148P.

**6.** Rekombinierte DNA nach Anspruch 4 in Form des Plasmids pBT 306.16, DSM 3149P.

**7.** Verfahren zur Herstellung des Mikroorganismus von Anspruch 1, **dadurch gekennzeichnet,** daß man DNA aus Pseudomonas putida
a) mit Eco R I limitiert verdaut und ein 5,8 Kb-Bruchstück gewinnt oder
b) mit Eco R1 und Pvu II spaltet und ein 2,2 Kb-Bruchstück gewinnt,
das nach a) oder b) erhaltene Bruchstück in einen mit dem bzw. den gleichen Restriktionsendonukleasen gespaltenen Vektor kloniert, diesen in einen für den gewählten Vektor geeigneten E. coli oder P. putida Stamm transformiert und die konstitutiv Creatinamidinohydrolase bildenden Klone isoliert.

**8.** Verfahren nach Anspruch 7, **dadurch gekennzeichnet,** daß man das Plasmid pBR 322 mit Eco R1 spaltet, in die Spaltstelle das 5,8 Kb-Fragment einführt und das erhaltene Plasmid in E. coli transformiert.

**9.** Verfahren nach Anspruch 7, **dadurch gekennzeichnet,** daß man pBR 322 mit Eco R1 und Pvu II spaltet, das 2,3 Kb-Fragment isoliert und mit dem 2,2 Kb Eco R1-Pvu II-Fragment aus P. putida verknüpft unter Bildung des Plasmids pBT 3-2, welches man in E. coli transformiert.

**10.** Verfahren nach einem der Ansprüche 7 bis 9, **dadurch gekennzeichnet,** daß man den transformierten E. coli nach Ampicillinresistenz selektiert.

**11.** Verfahren nach Anspruch 9 oder 10, **dadurch gekennzeichnet,** daß man die pBT 3-2 enthaltenden transformierten E. coli Zellen zum Zeitpunkt der Amplifikation des Plasmids mit Nitrosoguanidin behandelt, danach das Plasmid daraus isoliert, erneut in E. coli Zellen transformiert, gegebenenfalls diesen Zyklus wiederholt und aus den Klonen mit besonders ausgeprägter Creatinamidinohydrolase-Aktivität das Plasmid pBT 2a-1 gewinnt.

**12.** Verfahren nach Anspruch 11, **dadurch gekennzeichnet,** daß man das Plasmid RSF 1010 mit Pvu II spaltet, in die so erhaltene Spaltstelle, das aus dem Plasmid pACYC 177 mit Hae II geschnittene 1,4 Kb-DNA-Fragment ligiert, wobei man das Plasmid pBT 306.1 erhält, dieses mit Pvu I und Sma I spaltet, das gebildete 10 Kb-Bruchstück mit dem 2,8 Kb-Bruchstück ligiert, welches man durch Spaltung von pBT 2a-1 mit Pvu I und Pvu II erhält, und das Plasmid pBT 306.16 erhält.

**13.** Verfahren nach einem der Ansprüche 7 bis 12, **dadurch gekennzeichnet,** daß gebildete Klone der transformierten Stämme mit Agaroseplatten kontaktiert werden, welche Creatin, Sarcosinoxidase, Peroxidase und ein $H_2O_2$-Farbindikatorsystem gelöst enthalten und diejenigen Klone ausgewählt werden, welche die stärkste Färbung hervorrufen.

**14.** Verfahren nach Anspruch 13, **dadurch gekennzeichnet,** daß man als $H_2O_2$-Farbindikatorsystem 4-Aminoantipyrin in Kombination mit N-Ethyl-N-(sulfoethyl)-3-methylanilinsalz verwendet.

**Patentansprüche für folgenden Vertragsstaat : AT**

**1.** Verfahren zur Herstellung eines Mikroorganismus der Gattung E.coli oder Pseudomonas putida, welcher konstitutiv Creatinamidinohydrolase bildet,
**dadurch gekennzeichnet,**
daß man DNA aus Pseudomonas putida
a) mit Eco R I limitiert verdaut und ein 5,8 Kb-Bruchstück gewinnt oder
b) mit Eco R1 und Pvu II spaltet und ein 2,2 Kb-Bruchstück gewinnt,
das nach a) oder b) erhaltene Bruchstück in einen mit dem bzw. den gleichen Restriktionsendonukleasen gespaltenen Vektor kloniert, diesen in einen für den gewählten Vektor geeigneten E.coli oder P. putida Stamm transformiert und die konstitutiv Creatinamidinohydrolase bildenden Klone isoliert.

**2.** Verfahren nach Anspruch 1,
**dadurch gekennzeichnet,**
daß man das Plasmid pBR 322 mit Eco R1 spaltet, in die Spaltstelle das 5,8 Kb-Fragment einführt und das erhaltene Plasmid in E.coli transformiert.

**3.** Verfahren nach Anspruch 1,
**dadurch gekennzeichnet,**
daß man pBR 322 mit Eco R1 und Pvu II spaltet, das 2,3 Kb-Fragment isoliert und mit dem 2,2 Kb Eco R1-Pvu II-Fragment aus P. putida verknüpft unter Bildung des Plasmids pBT 3-2, welches man in E.coli transformiert.

**4.** Verfahren nach einem der Ansprüche 1 bis 3,
**dadurch gekennzeichnet,**
daß man den transformierten E.coli nach Ampicillinresistenz selektiert.

**5.** Verfahren nach Anspruch 3 oder 4,
**dadurch gekennzeichnet,**
daß man die pBT 3-2 enthaltenden transformierten E.coli Zellen zum Zeitpunkt der Amplifikation des Plasmids mit Nitrosoguanidin behandelt, danach das Plasmid daraus isoliert, erneut in E.coli Zellen transformiert, gegebenenfalls diesen Zyklus wiederholt und aus den Klonen mit besonders ausgeprägter Creatinamidinohydrolase-Aktivität das Plasmid pBT 2a-1 gewinnt.

**6.** Verfahren nach Anspruch 5,
**dadurch gekennzeichnet,**
daß man das Plasmid RSF 1010 mit Pvu II spaltet, in die so erhaltene Spaltstelle, das aus dem Plasmid pACYC 177 mit Hae II geschnittene 1,4 Kb-DNA-Fragment ligiert, wobei man das Plasmid pBT 306.1 erhält, dieses mit Pvu I und Sma I spaltet, das gebildete 10 Kb-Bruchstück mit dem 2,8 Kb-Bruchstück ligiert, welches man durch Spaltung von pBT 2a-1 mit Pvu I und Pvu II erhält, und das Plasmid pBT 306.16 erhält.

**7.** Verfahren nach einem der Ansprüche 1 bis 6,
**dadurch gekennzeichnet,**

daß gebildete Klone der transformierten Stämme mit Agaroseplatten kontaktiert werden, welche Creatin, Sarcosinoxidase, Peroxidase und ein $H_2O_2$-Farbindikatorsystem gelöst enthalten und diejenigen Klone ausgewählt werden, welche die stärkste Färbung hervorrufen.

**8.** Verfahren nach Anspruch 7,
**dadurch gekennzeichnet,**
daß man als $H_2O_2$-Farbindikatorsystem 4-Aminoantipyrin in Kombination mit N-Ethyl-N-(sulfoethyl)-3-methylanilinsalz verwendet.

## Claims

### Claims for the following Contracting States : BE, CH, DE, FR, GB, IT, LI, LU, NL, SE

**1.** Micro-organism of the species E. coli or Pseudomonas putida, characterised in that it forms creatinamidinohydrolase constitutively and is obtainable by transformation with a recombinant plasmid which contains creatinamidinohydrolase-coding DNA which has been obtained from Pseudomonas putida DNA

a) by limited digestion with Eco RI and isolation of a 5.8 kb fragment or

b) by cleavage with Eco RI and Pvu II and isolation of a 2.2 kb fragment.

**2.** Micro-organism of the species E. coli according to claim 1, characterised in that it contains the plasmid pBT 2a-1, DSM 3148P

**3.** Micro-organisms of the species E. coli or Pseudomonas putida according to claim 1, characterised in that it contains the plasmid pBT 306.16, DSM 3149P.

**4.** Recombinant DNA, characterised in that it contains the base sequence 1 to 1212 coding for the creatine splitting protein of the given amino acid sequence 1 to 403.

```
MetGlnMetProLysThrLeuArgIleArgAsnGlyAspLysValArgSerThrPheSer
ATGCAAATGCCCAAGACGCTCCGCATCCGTAACGGCGACAAGGTTCGCTCCACCTTCTCC

AlaGlnGluTyrAlaAsnArgGlnAlaArgLeuArgAlaHisLeuAlaAlaGluAsnIle
GCCCAGGAATACGCCAATCGCCAAGCCAGGCTGCGCGCCCACCTGGCGGCGGAGAACATC

AspAlaAlaIlePheZhrSerTyrHisAsnIleAsnTyrTyrSerAspPheLeuTyrCys
GACGCCGCGATCTTCACCTCGTACCACAACATCAACTACTACTCCGACTTCCTCTACTGC
```

SerPheGlyArgProTyrAlaLeuValValThrHisAspAspValIleSerIleSerAla
TCCTTCGGCCGCCCCTACGCGTTGGTGGTGACCCACGACGACGTCATCAGCATCAGCGCC

AsnIleAspGlyGlyGlnProTrpArgArgThrValGlyThrAspAsnIleValTyrThr
AACATCGACGGCGGCCAGCCGTGGCGCCGCACCGTCGGCACCGACAACATCGTCTACACC

AspTrpGlnArgAspAsnTyrPheAlaAlaIleGlnGlnAlaLeuProLysAlaArgArg
GACTGGCAGCGCGATAACTACTTCGCCGCCATCCAGCAGGCGTTGCCGAAGGCCCGCCGC

IleGlyIleGluHisAspHisLeuAsnLeuGlnAsnArgAspLysLeuAlaAlaArgTyr
ATCGGCATCGAACATGACCACCTGAACCTGCAGAACCGCGACAAGCTGGCCGCGCGCTAT

ProAspAlaGluLeuValAspValAlaAlaAlaCysMetArgMetArgMetIleLysSer
CCGGACGCCGAGCTGGTGGACGTGGCCGCCGCCTGCATGCGTATGCGCATGATCAAATCC

AlaGluGluHisValMetIleArgHisGlyAlaArgIleAlaAspIleGlyGlyAlaAla
GCCGAAGAGCACGTGATGATCCGCCACGGCGCGCGCATCGCCGACATCGGTGGTGCGGCG

ValValGluAlaLeuGlyAspGlnValProGluTyrGluValAlaLeuHisAlaThrGln
GTGGTCGAAGCCCTGGGCGACCAGGTACCGGAATACGAAGTGGCGCTGCATGCCACCCAG

AlaMetValArgAlaIleAlaAspThrPheGluAspValGluLeuMetAspThrTrpThr
GCCATGGTCCGCGCCATTGCCGATACCTTCGAGGACGTGGAGCTGATGGATACCTGGACC

TrpPheGlnSerGlyIleAsnThrAspGlyAlaHisAsnProValThrThrArgLysVal
TGGTTCCAGTCCGGCATCAACACCGACGGCGCGCACAACCCGGTGACCACCCGCAAGGTG

AsnLysGlyAspIleLeuSerLeuAsnCysPheProMetIleAlaGlyTyrTyrThrAla
AACAAGGGCGACATCCTCAGCCTCAACTGCTTCCCGATGATCGCCGGCTACTACACCGCG

LeuGluArgThrLeuPheLeuAspHisCysSerAspAspHisLeuArgLeuTrpGlnVal
TTGGAGCGCACCCTGTTCCTCGACCACTGCTCGGACGACCACCTGCGTCTGTGGCAGGTC

AsnValGluValHisGluAlaGlyLeuLysLeuIleLysProGlyAlaArgCysSerAsp
AACGTCGAGGTGCATGAAGCCGGCCTGAAGCTGATCAAGCCCGGTGCGCGTTGCAGCGAT

IleAlaArgGluLeuAsnGluIlePheLeuLysHisAspValLeuGlnTyrArgThrPhe
ATCGCCCGCGAGCTGAACGAGATCTTCCTCAAGCACGACGTGCTGCAGTACCGCACCTTC

GlyTyrGlyHisSerPheGlyThrLeuSerHisTyrTyrGlyArgGluAlaGlyLeuGlu
GGCTACGGCCACTCCTTCGGCACGCTCAGCCACTACTACGGCCGCGAGGCCGGGTTGGAA

LeuArgGluAspIleAspThrValLeuGluProGlyMetValValSerMetGluProMet
CTGCGCGAGGACATCGACACCGTGCTGGAGCCGGGCATGGTGGTGTCGATGGAGCCGATG

IleMetLeuProGluGlyLeuProGlyAlaGlyGlyTyrArgGluHisAspIleLeuIle
ATCATGCTGCCGGAAGGCCTGCCGGGCGCCGGTGGCTATCGCGAGCACGACATCCTGATC

ValAsnGluAsnGlyAlaGluAsnIleThrLysPheProTyrGlyProGluLysAsnIle
GTCAACGAGAACGGTGCCGAGAACATCACCAAGTTCCCCTACGGCCCGGAGAAAAACATC

IleArgLys
ATCCGCAAATGA

or an equivalent coding according to the genetic code for the same amino acid sequence.

**5.** Recombinant DNA according to claim 4 in the form of the plasmid pBT 2a-1, DSM 3148P.

**6.** Recombinant DNA according to claim 4 in the form of the plasmid pBT 306.16, DSM 3149P.

**7.** Process for the production of the micro-organism according to claim 1, characterised in that DNA from Pseudomonas putida is
   a) limitedly digested with Eco R I and a 5.8 Kb fragment is obtained or
   b) cleaved with Eco R I and Pvu II and a 2.2 Kb fragment is obtained,
the fragment obtained according to a) or b) is cloned into a vector cleaved with the same restriction endonuclease(s), this is transformed into an E. coli or P. putida strain suitable for the selected vector and the clones constitutively forming creatinamidinohydrolase isolated.

**8.** Process according to claim 7, characterised in that one cleaves the plasmid pBR 322 with Eco RI, introduces the 5.8 Kb fragment into the cleavage position and transforms the plasmid obtained into E. coli.

**9.** Process according to claim 7, characterised in that one cleaves pBR 322 with Eco RI and Pvu II, isolates the 2.3 Kb fragment and links with the 2.2 Kb Eco RI-Pvu II fragment from P. putida with the formation of the plasmid pBT 3-2, which one transforms into E. coli.

**10.** Process according to one of claims 7 to 9, characterised in that one selects the transformed E. coli according to ampicillin resistance.

**11.** Process according to claim 9 or 10, characterised in that one treats the pBT 3-2-containing transformed E. coli cells at the point of time of the amplification of the plasmid with nitrosoguanidine, thereafter isolates the plasmid therefrom, again transformed into E. coli cells, possibly repeats this cycle and obtains the plasmid pBT 2a-1 from the clones with especially marked creatinamidinohydrolase activity.

**12.** Process according to claim 11, characterised in that one cleaves the plasmid RSF 1010 with Pvu II, ligates into the so obtained cleavage position the 1.4 Kb DNA fragment cleaved from the plasmid pACYC 177 with Hae II, whereby one obtains the plasmid pBT 306.1, cleaves this with Pvu I and Sma I, ligates the 10 Kb fragment formed with the 2.8 Kb fragment, which one obtains by cleavage of pBT 2a-1 with Pvu I and Pvu II and obtains the plasmid pBT 306.16.

**13.** Process according to one of claims 7 to 12, characterised in that clones formed of the transformed strains are contacted with agarose plates which contain dissolved creatine, sarcosine oxidase, peroxidase and an $H_2O_2$ colour indicator system and those clones are selected which bring about the strongest coloration.

**14.** Process according to claim 13, characterised in that, as $H_2O_2$ colour indicator system, one uses 4-aminoantipyrine in combination with N-ethyl-N-(sulphoethyl)-3-methylaniline salt.

**Claims for following Contracting State : AT**

**1.** Process for the production of a micro-organism of the species E. coli or Pseudomonas putida which constitutively forms creatinamidinohydrolase, characterised in that DNA from Pseudomonas putida
a) is limitedly digested with Eco RI and a 5.8 Kb fragment is obtained or
b) is cleaved with Eco RI and Pvu II and a 2.2 Kb fragment is obtained,
the fragment obtained according to a) or b) is cloned into a vector cleaved with the same restriction endonuclease(s), this is transformed into an E. coli or P. putida strain suitable for the selected vector and the constitutively creatinamidinohydrolase-forming clones isolated.

**2.** Process according to claim 1, characterised in that one cleaves the plasmid pBR 322 with Eco RI, introduces the 5.8 Kb fragment into the cleavage position and transforms the plasmid obtained into E. coli.

**3.** Process according to claim 1, characterised in that one cleaves pBR 322 with Eco RI and Pvu II, isolates the 2.3 Kb fragment and links with the 2.2 Kb Eco RI-Pvu II fragment from P. putida with the formation of the plasmid pBT 3-2 which one transforms into E. coli.

**4.** Process according to one of claims 1 to 3, characterised in that one selects the transformed E. coli according to ampicillin resistance.

**5.** Process according to claim 3 or 4, characterised in that one treats the pBT 3-2-containing transformed E. coli cells at the point of time of the amplification of the plasmid with nitrosoguanidine, thereafter isolates the plasmid therefrom, again transforms into E. coli cells, possibly repeats this cycle and obtains the plasmid pBT 2a-1 from the clones with especially marked creatinamidinohydrolase activity.

**6.** Process according to claim 5, characterised in that one cleaves the plasmid RSF 1010 with Pvu II, ligates into the so obtained cleavage position the 1.4 Kb DNA fragment split from the plasmid pACYC 177 cleaved with Hae II, whereby one obtains the plasmid pBT 306.1, cleaves this with Pvu I and Sma I, ligates the 10 Kb fragment formed with the 2.8 Kb fragment which one obtains by cleavage of pBT 2a-1 with Pvu I and Pvu II, and obtains the plasmid pBT 306.16.

**7.** Process according to one of claims 1 to 6, characterised in that clones formed of the transformed strains are contacted with agarose plates which contain creatine, sarcosine oxidase, peroxidase and an $H_2O_2$ colour indicator system and those clones are selected which bring about the strongest coloration.

**8.** Process according to claim 7, characterised in that, as $H_2O_2$ colour indicator system, one uses 4-aminoantipyrine in combination with N-ethyl-N-(sulphoethyl)-3-methylaniline salt.

**Revendications**

**Revendications pour les Etats contractants suivants : BE, CH, DE, FR, GB, IT, LI, LU, NL, SE**

**1.** Microorganisme du genre E. coli ou Pseudomonas putida, caractérisé en ce qu'il forme de la créatinamidinohydrolase de manière constitutive et qu'il peut être obtenu par transformation avec un

plasmide recombinant qui contient de l'ADN codant pour la créatinamidinohydrolase qui a été obtenu à partir d'ADN de Pseudomonas putida

a) par digestion limitée avec Eco RI et isolement d'un fragment de 5,8 kb ou

b) par coupure avec Eco RI et Pvu II et isolement d'un fragment de 2,2 kb.

**2.** Microorganisme du genre E.coli selon la revendication 1, caractérisé en ce qu'il contient le plasmide pBT 2a-1, DSM 3148P.

**3.** Microorganisme du genre E. coli ou Pseudomonas putida selon la revendication 1, caractérisé en ce qu'il contient le plasmide pBT 306.16, DSM 3149P.

**4.** ADN recombiné, caractérisé en ce qu'il contient la séquence de bases 1 à 1212 qui code pour la protéine coupant la créatine de séquence d'acides aminés 1 à 403

MetGlnMetProLysThrLeuArgIleArgAsnGlyAspLysValArgSerThrPheSer
ATGCAAATGCCCAAGACGCTCCGCATCCGTAACGGCGACAAGGTTCGCTCCACCTTCTCC

AlaGlnGluTyrAlaAsnArgGlnAlaArgLeuArgAlaHisLeuAlaAlaGluAsnIle
GCCCAGGAATACGCCAATCGCCAAGCCAGGCTGCGCGCCCACCTGGCGGCGGAGAACATC

AspAlaAlaIlePheZhrSerTyrHisAsnIleAsnTyrTyrSerAspPheLeuTyrCys
GACGCCGCGATCTTCACCTCGTACCACAACATCAACTACTACTCCGACTTCCTCTACTGC

SerPheGlyArgProTyrAlaLeuValValThrHisAspAspValIleSerIleSerAla
TCCTTCGGCCGCCCCTACGCGTTGGTGGTGACCCACGACGACGTCATCAGCATCAGCGCC

AsnIleAspGlyGlyGlnProTrpArgArgThrValGlyThrAspAsnIleValTyrThr
AACATCGACGGCGGCCAGCCGTGGCGCCGCACCGTCGGCACCGACAACATCGTCTACACC

AspTrpGlnArgAspAsnTyrPheAlaAlaIleGlnGlnAlaLeuProLysAlaArgArg
GACTGGCAGCGCGATAACTACTTCGCCGCCATCCAGCAGGCGTTGCCGAAGGCCCGCCGC

IleGlyIleGluHisAspHisLeuAsnLeuGlnAsnArgAspLysLeuAlaAlaArgTyr
ATCGGCATCGAACATGACCACCTGAACCTGCAGAACCGCGACAAGCTGGCCGCGCGCTAT

ProAspAlaGluLeuValAspValAlaAlaAlaCysMetArgMetArgMetIleLysSer
CCGGACGCCGAGCTGGTGGACGTGGCCGCCGCCTGCATGCGTATGCGCATGATCAAATCC

AlaGluGluHisValMetIleArgHisGlyAlaArgIleAlaAspIleGlyGlyAlaAla
GCCGAAGAGCACGTGATGATCCGCCACGGCGCGCGCATCGCCGACATCGGTGGTGCGGCG

ValValGluAlaLeuGlyAspGlnValProGluTyrGluValAlaLeuHisAlaThrGln
GTGGTCGAAGCCCTGGGCGACCAGGTACCGGAATACGAAGTGGCGCTGCATGCCACCCAG

AlaMetValArgAlaIleAlaAspThrPheGluAspValGluLeuMetAspThrTrpThr
GCCATGGTCCGCGCCATTGCCGATACCTTCGAGGACGTGGAGCTGATGGATACCTGGACC

TrpPheGlnSerGlyIleAsnThrAspGlyAlaHisAsnProValThrThrArgLysVal
TGGTTCCAGTCCGGCATCAACACCGACGGCGCGCACAACCCGGTGACCACCCGCAAGGTG

AsnLysGlyAspIleLeuSerLeuAsnCysPheProMetIleAlaGlyTyrTyrThrAla
AACAAGGGCGACATCCTCAGCCTCAACTGCTTCCCGATGATCGCCGGCTACTACACCGCG

LeuGluArgThrLeuPheLeuAspHisCysSerAspAspHisLeuArgLeuTrpGlnVal
TTGGAGCGCACCCTGTTCCTCGACCACTGCTCGGACGACCACCTGCGTCTGTGGCAGGTC

AsnValGluValHisGluAlaGlyLeuLysLeuIleLysProGlyAlaArgCysSerAsp
AACGTCGAGGTGCATGAAGCCGGCCTGAAGCTGATCAAGCCCGGTGCGCGTTGCAGCGAT

IleAlaArgGluLeuAsnGluIlePheLeuLysHisAspValLeuGlnTyrArgThrPhe
ATCGCCCGCGAGCTGAACGAGATCTTCCTCAAGCACGACGTGCTGCAGTACCGCACCTTC

GlyTyrGlyHisSerPheGlyThrLeuSerHisTyrTyrGlyArgGluAlaGlyLeuGlu
GGCTACGGCCACTCCTTCGGCACGCTCAGCCACTACTACGGCCGCGAGGCCGGGTTGGAA

LeuArgGluAspIleAspThrValLeuGluProGlyMetValValSerMetGluProMet
CTGCGCGAGGACATCGACACCGTGCTGGAGCCGGGCATGGTGGTGTCGATGGAGCCGATG

IleMetLeuProGluGlyLeuProGlyAlaGlyGlyTyrArgGluHisAspIleLeuIle
ATCATGCTGCCGGAAGGCCTGCCGGGCGCCGGTGGCTATCGCGAGCACGACATCCTGATC

ValAsnGluAsnGlyAlaGluAsnIleThrLysPheProTyrGlyProGluLysAsnIle
GTCAACGAGAACGGTGCCGAGAACATCACCAAGTTCCCCTACGGCCCGGAGAAAAACATC

IleArgLys
ATCCGCAAATGA

ou un équivalent de celle-ci codant pour la même séquence d'acides aminés selon le code génétique.

**5.** ADN recombiné selon la revendication 4 sous forme du plasmide pBT 2a-1, DSM 3148P.

**6.** ADN recombiné selon la revendication 4 sous forme du plasmide pBT 306.16, DSM 3149P.

**7.** Procédé de préparation du microorganisme selon la revendication 1, caractérisé en ce que
a) on digère de manière limitée l'ADN de Pseudomonas putida avec Eco RI et on obtient un fragment de 5,8 kb ou
b) on coupe l'ADN de Pseudomonas putida avec Eco RI et Pvu II et on obtient un fragment de 2,2 kb,
on clone le fragment obtenu selon a) ou b) dans un vecteur coupé par la même ou les mêmes endonucléases de restriction, on le transforme dans une souche de E. coli ou de P. putida convenant pour le vecteur choisi et on isole les clones formant de la créatinamidinohydrolase de manière constitutive.

**8.** Procédé selon la revendication 7, caractérisé en ce que l'on coupe le plasmide pBR 322 avec Eco RI, on introduit le fragment de 5,8 kb dans le site de coupure et on transforme le plasmide obtenu dans E. coli.

**9.** Procédé selon la revendication 7, caractérisé en ce que l'on coupe pBR 322 avec Eco RI et Pvu II, on isole le fragment de 2,3 kb et on le relie au fragment Eco RI-Pvu II de 2,2 kb provenant de P. putida pour former le plasmide pBT 3-2 que l'on transforme dans E. coli.

**10.** Procédé selon l'une des revendications 7 à 9, caractérisé en ce que l'on sélectionne E. coli transformé selon la résistance à l'ampicilline.

**11.** Procédé selon la revendication 9 ou 10, caractérisé en ce que, au moment de l'amplification du plasmide, on traite à la nitrosoguanidine les cellules de E. coli transformées contenant pBT 3-2, puis on

en isole le plasmide, on le transforme de nouveau dans des cellules de E. coli, on répète éventuellement ce cycle et on obtient à partir des clones à activité de créatinamidinohydrolase particulièrement marquée le plasmide pBT 2a-1.

**12.** Procédé selon la revendication 11, caractérisé en ce que l'on coupe le plasmide RSF 1010 avec Pvu II, on ligature dans le site de coupure ainsi obtenu le fragment d'ADN de 1,4 kb coupé avec Hae II dans le plasmide pACYC 177 pour obtenir le plasmide pBT 306.1, on coupe celui-ci avec Pvu I et Sma I, on ligature le fragment de 10 kb formé avec le fragment de 2,8 kb que l'on obtient par coupure de pBT 2a-1 avec Pvu I et Pvu II, et on obtient le plasmide pBT 306.16.

**13.** Procédé selon l'une des revendications 7 à 12, caractérisé en ce l'on met les clones formés des souches transformées en contact avec des plaques d'agarose qui contiennent à l'état dissous de la créatine, de la sarcosinoxydase, de la peroxydase et un système d'indicateur coloré à $H_2O_2$ et on choisit les clones qui provoquent la plus forte coloration.

**14.** Procédé selon la revendication 13, caractérisé en ce que l'on utilise comme système d'indicateur coloré à $H_2O_2$ la 4-aminoantipyrine en combinaison avec un sel de N-éthyl-N-(sulfoéthyl)-3-méthylaniline.

**Revendications pour l'Etat contractant suivant : AT**

**1.** Procédé de préparation d'un microorganisme du genre E.coli ou Pseudomonas putida qui forme de la créatinamidinohydrolase de manière constitutive, caractérisé en ce que
a) on digère de manière limitée l'ADN de Pseudomonas putida avec Eco RI et on obtient un fragment de 5,8 kb ou
b) on coupe l'ADN de Pseudomonas putida avec Eco RI et Pvu II et on obtient un fragment de 2,2 kb,
on clone le fragment obtenu selon a) ou b) dans un vecteur coupé par la même ou les mêmes endonucléases de restriction, on le transforme dans une souche de E. coli ou de P. putida convenant pour le vecteur choisi et on isole les clones formant de la créatinamidinohydrolase de manière constitutive.

**2.** Procédé selon la revendication 1, caractérisé en ce que l'on coupe le plasmide pBR 322 avec Eco RI, on introduit le fragment de 5,8 kb dans le site de coupure et on transforme le plasmide obtenu dans E. coli.

**3.** Procédé selon la revendication 1, caractérisé en ce que l'on coupe pBR 322 avec Eco RI et Pvu II, on isole le fragment de 2,3 kb et on le relie au fragment Eco RI-Pvu II de 2,2 kb provenant de P. putida pour former le plasmide pBT 3-2 que l'on transforme dans E. coli.

**4.** Procédé selon l'une des revendications 1 à 3, caractérisé en ce que l'on sélectionne E. coli transformé selon la résistance à l'ampicilline.

**5.** Procédé selon la revendication 3 ou 4, caractérisé en ce que, au moment de l'amplification du plasmide, on traite à la nitrosoguanidine les cellules de E. coli transformées contenant pBT 3-2, puis on en isole le plasmide, on le transforme de nouveau dans des cellules de E. coli, on répète éventuellement ce cycle et on obtient à partir des clones à activité de créatinamidinohydrolase particulièrement marquée le plasmide pBT 2a-1.

**6.** Procédé selon la revendication 5, caractérisé en ce que l'on coupe le plasmide RSF 1010 avec Pvu II, on ligature dans le site de coupure ainsi obtenu le fragment d'ADN de 1,4 Kb coupé avec Hae II dans le plasmide pACYC 177 pour obtenir le plasmide pBT 306.1, on coupe celui-ci avec Pvu I et Sma I, on ligature le fragment de 10 kb formé avec le fragment de 2,8 kb que l'on obtient par coupure de pBT 2a-1 avec Pvu I et Pvu II, et on obtient le plasmide pBT 306.16.

**7.** Procédé selon l'une des revendications 1 à 6, caractérisé en ce l'on met les clones formés des souches transformées en contact avec des plaques d'agarose qui contiennent à l'état dissous de la créatine, de la sarcosinoxydase, de la peroxydase et un système d'indicateur coloré à $H_2O_2$ et on

choisit les clones qui provoquent la plus forte coloration.

**8.** Procédé selon la revendication 7, caractérisé en ce que l'on utilise conme système d'indicateur coloré à $H_2O_2$ la 4-aminoantipyrine en combinaison avec un sel de N-éthyl-N-(sulfoéthyl)-3-méthylaniline.

Fig.1

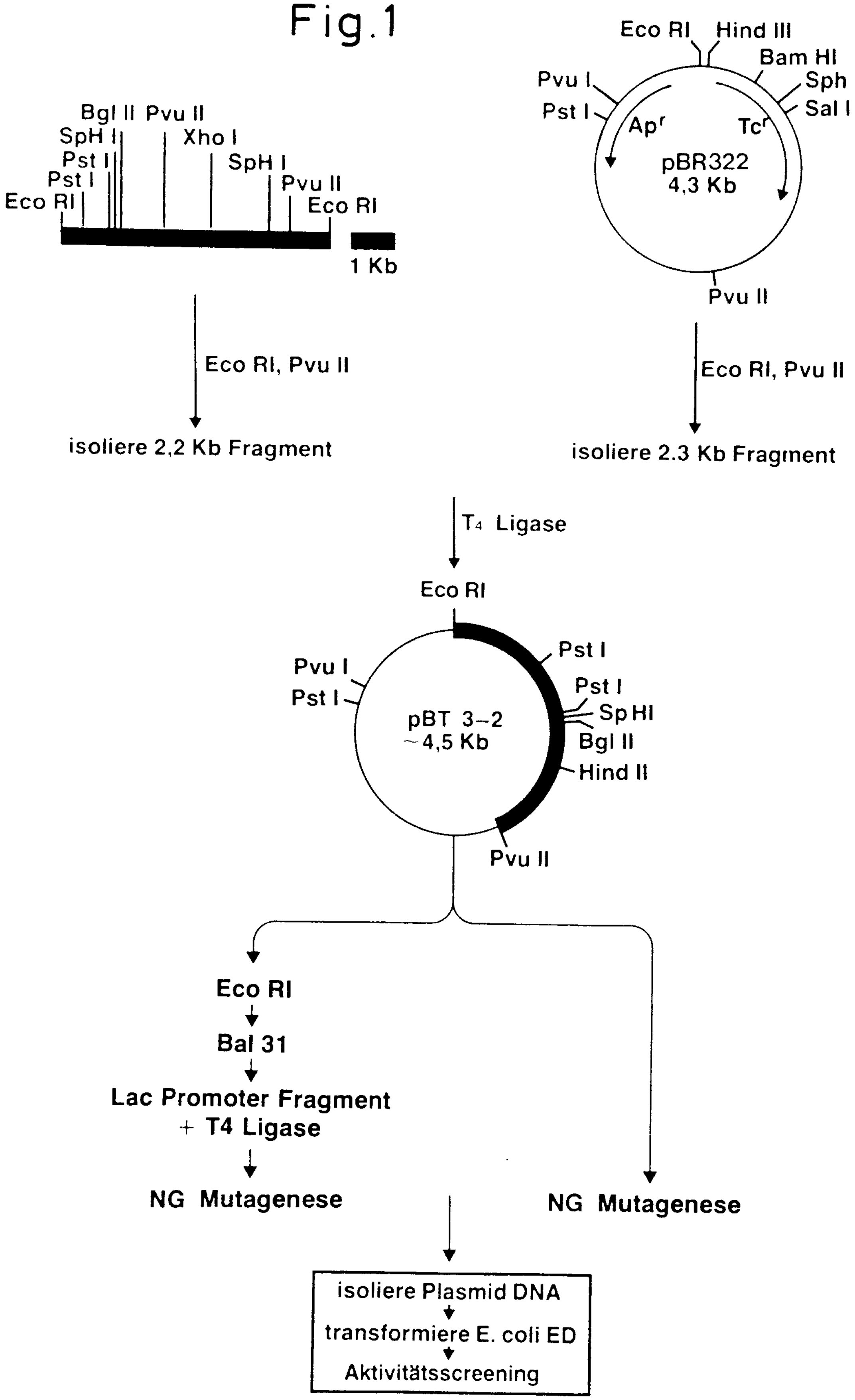
Bgl II Pvu II
SpH I
Xho I
Pst I
SpH I
Pst I
Pvu II
Eco RI
Eco RI
1 Kb
Eco RI Hind III
Bam HI
Pvu I
Sph I
Pst I
Sal I
Ap^r
Tc^r
pBR322
4,3 Kb
Pvu II
Eco RI, Pvu II
isoliere 2,2 Kb Fragment
Eco RI, Pvu II
isoliere 2.3 Kb Fragment
T4 Ligase
Eco RI
Pst I
Pvu I
Pst I
Pst I
SpHI
pBT 3-2
~4,5 Kb
Bgl II
Hind II
Pvu II
Eco RI
Bal 31
Lac Promoter Fragment
+ T4 Ligase
NG Mutagenese
NG Mutagenese
isoliere Plasmid DNA
transformiere E. coli ED
Aktivitätsscreening

Fig. 2

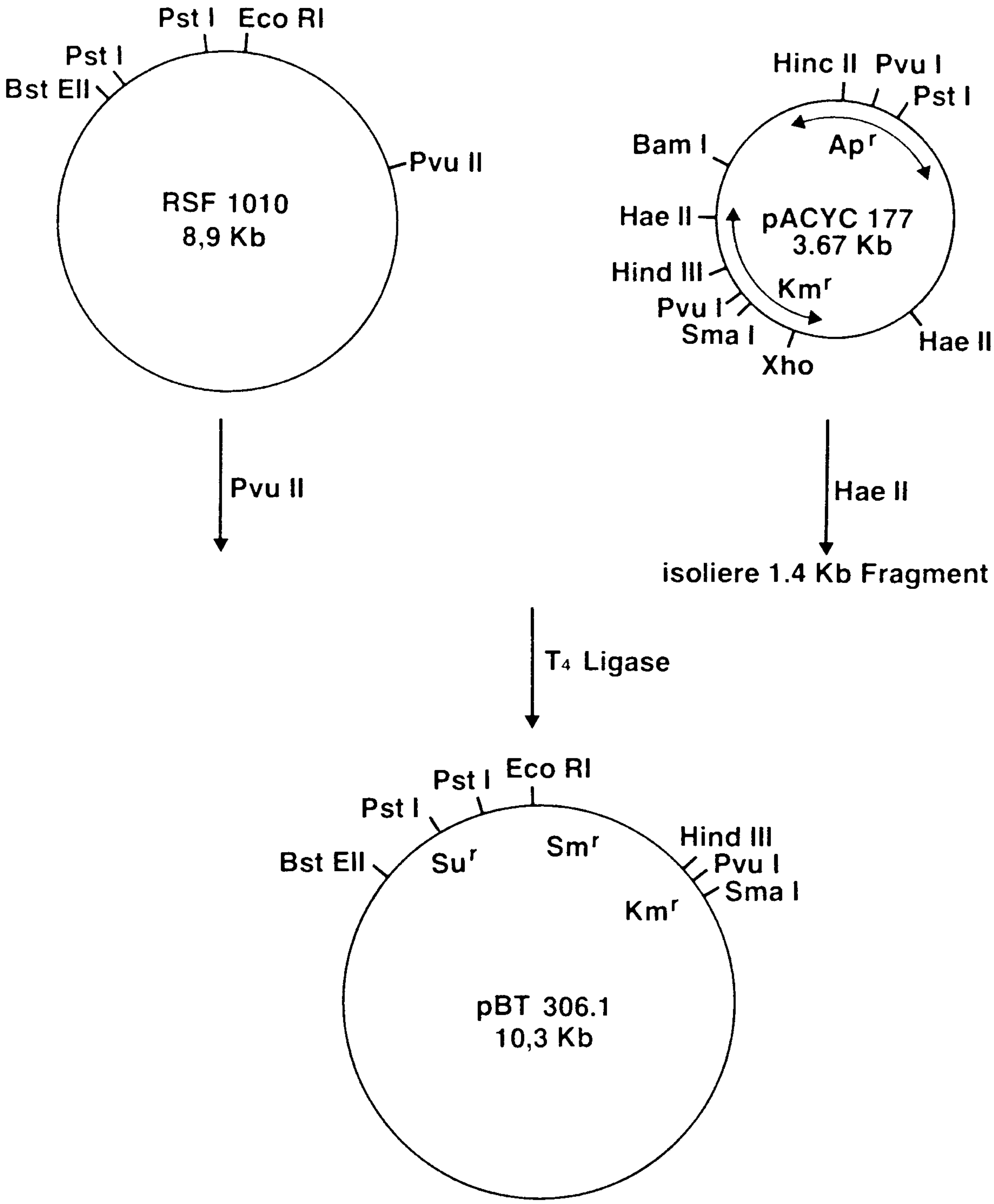
Pst I
Eco RI
Pst I
Bst EII
Pvu II
RSF 1010
8,9 Kb
Hinc II
Pvu I
Pst I
Bam I
Ap^r
Hae II
pACYC 177
3.67 Kb
Hind III
Km^r
Pvu I
Sma I
Xho
Hae II
Pvu II
Hae II
isoliere 1.4 Kb Fragment
T4 Ligase
Pst I
Eco RI
Pst I
Bst EII
Su^r
Sm^r
Hind III
Pvu I
Sma I
Km^r
pBT 306.1
10,3 Kb

Fig. 3

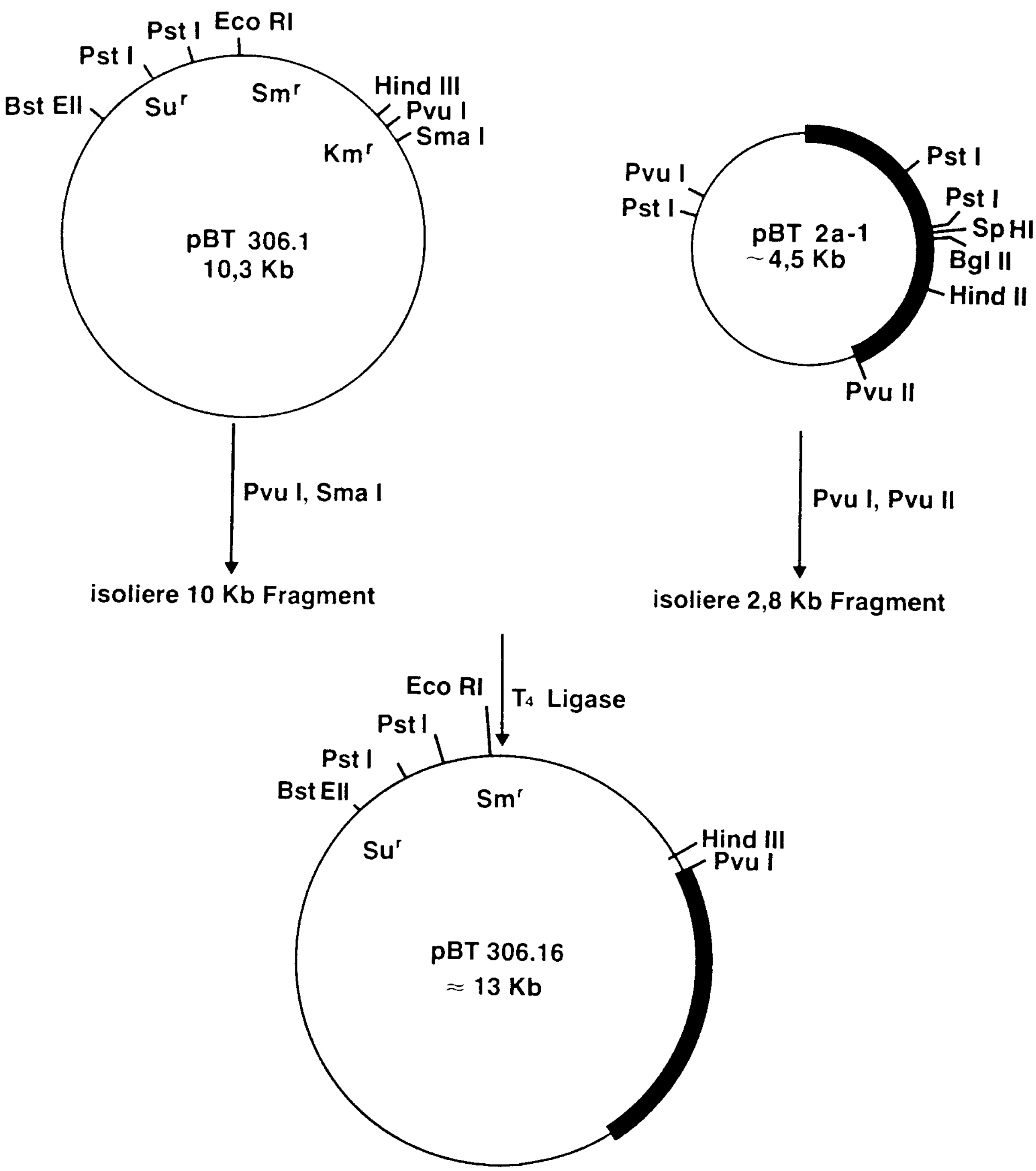
Pst I
Eco RI
Pst I
Bst EII
Su^r
Sm^r
Hind III
Pvu I
Sma I
Km^r
pBT 306.1
10,3 Kb
Pvu I, Sma I
isoliere 10 Kb Fragment
Pst I
Pvu I
Pst I
Pst I
Sp HI
Bgl II
Hind II
pBT 2a-1
~4,5 Kb
Pvu II
Pvu I, Pvu II
isoliere 2,8 Kb Fragment
T4 Ligase
Eco RI
Pst I
Pst I
Bst EII
Sm^r
Su^r
Hind III
Pvu I
pBT 306.16
≈ 13 Kb

Fig. 4

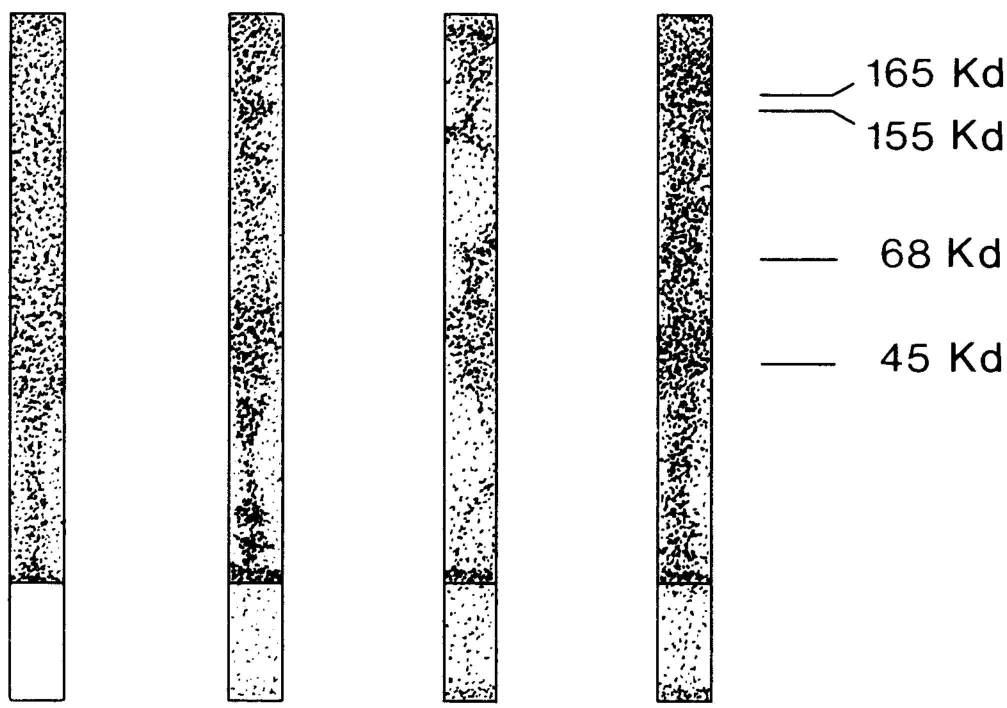

# FIG.5

```
  1*  MetGlnMetProLysThrLeuArgIleArgAsnGlyAspLysValArgSerThrPheSer   20
  1   ATGCAAATGCCCAAGACGCTCCGCATCCGTAACGGCGACAAGGTTCGCTCCACCTTCTCC   60
               *         *         *         *         *         *

 21   AlaGlnGluTyrAlaAsnArgGlnAlaArgLeuArgAlaHisLeuAlaAlaGluAsnIle   40
 61   GCCCAGGAATACGCCAATCGCCAAGCCAGGCTGCGCGCCCACCTGGCGGCGGAGAACATC  120
               *         *         *         *         *         *

 41   AspAlaAlaIlePheThrSerTyrHisAsnIleAsnTyrTyrSerAspPheLeuTyrCys   60
121   GACGCCGCGATCTTCACCTCGTACCACAACATCAACTACTACTCCGACTTCCTCTACTGC  180
               *         *         *         *         *         *

 61   SerPheGlyArgProTyrAlaLeuValValThrHisAspAspValIleSerIleSerAla   80
181   TCCTTCGGCCGCCCCTACGCGTTGGTGGTGACCCACGACGACGTCATCAGCATCAGCGCC  240
               *         *         *         *         *         *

 81   AsnIleAspGlyGlyGlnProTrpArgArgThrValGlyThrAspAsnIleValTyrThr  100
241   AACATCGACGGCGGCCAGCCGTGGCGCCGCACCGTCGGCACCGACAACATCGTCTACACC  300
               *         *         *         *         *         *

101   AspTrpGlnArgAspAsnTyrPheAlaAlaIleGlnGlnAlaLeuProLysAlaArgArg  120
301   GACTGGCAGCGCGATAACTACTTCGCCGCCATCCAGCAGGCGTTGCCGAAGGCCCGCCGC  360
               *         *         *         *         *         *

121   IleGlyIleGluHisAspHisLeuAsnLeuGlnAsnArgAspLysLeuAlaAlaArgTyr  140
361   ATCGGCATCGAACATGACCACCTGAACCTGCAGAACCGCGACAAGCTGGCCGCGCGCTAT  420
               *         *         *         *         *         *

141   ProAspAlaGluLeuValAspValAlaAlaAlaCysMetArgMetArgMetIleLysSer  160
421   CCGGACGCCGAGCTGGTGGACGTGGCCGCCGCCTGCATGCGTATGCGCATGATCAAATCC  480
               *         *         *         *         *         *

161   AlaGluGluHisValMetIleArgHisGlyAlaArgIleAlaAspIleGlyGlyAlaAla  180
481   GCCGAAGAGCACGTGATGATCCGCCACGGCGCGCGCATCGCCGACATCGGTGGTGCGGCG  540
               *         *         *         *         *         *

181   ValValGluAlaLeuGlyAspGlnValProGluTyrGlyValAlaLeuHisAlaThrGln  200
541   GTGGTCGAAGCCCTGGGCGACCAGGTACCGGAATACGAAGTGGCGCTGCATGCCACCCAG  600
               *         *         *         *         *         *

201   AlaMetValArgAlaIleAlaAspThrPheGluAspValGluLeuMetAspThrTrpThr  220
601   GCCATGGTCCGCGCCATTGCCGATACCTTCGAGGACGTGGAGCTGATGGATACCTGGACC  660
               *         *         *         *         *         *

221   TrpPheGlnSerGlyIleAsnThrAspGlyAlaHisAsnProValThrThrArgLysVal  240
661   TGGTTCCAGTCCGGCATCAACACCGACGGCGCGCACAACCCGGTGACCACCCGCAAGGTG  720
               *         *         *         *         *         *
```

```
 241   AsnLysGlyAspIleLeuSerLeuAsnCysPheProMetIleAlaGlyTyrTyrThrAla    260
 721   AACAAGGGCGACATCCTCAGCCTCAACTGCTTCCCGATGATCGCCGGCTACTACACCGCG    780
                   *         *         *         *         *         *

 261   LeuGluArgThrLeuPheLeuAspHisCysSerAspAspHisLeuArgLeuTrpGlnVal    280
 781   TTGGAGCGCACCCTGTTCCTCGACCACTGCTCGGACGACCACCTGCGTCTGTGGCAGGTC    840
                   *         *         *         *         *         *

 281   AsnValGluValHisGluAlaGlyLeuLysLeuIleLysProGlyAlaArgCysSerAsp    300
 841   AACGTCGAGGTGCATGAAGCCGGCCTGAAGCTGATCAAGCCCGGTGCGCGTTGCAGCGAT    900
                   *         *         *         *         *         *

 301   IleAlaArgGluLeuAsnGluIlePheLeuLysHisAspValLeuGlnTyrArgThrPhe    320
 901   ATCGCCCGCGAGCTGAACGAGATCTTCCTCAAGCACGACGTGCTGCAGTACCGCACCTTC    960
                   *         *         *         *         *         *

 321   GlyTyrGlyHisSerPheGlyThrLeuSerHisTyrTyrGlyArgGluAlaGlyLeuGlu    340
 961   GGCTACGGCCACTCCTTCGGCACGCTCAGCCACTACTACGGCCGCGAGGCCGGGTTGGAA   1020
                   *         *         *         *         *         *

 341   LeuArgGluAspIleAspThrValLeuGluProGlyMetValValSerMetGluProMet    360
1021   CTGCGCGAGGACATCGACACCGTGCTGGAGCCGGGCATGGTGGTGTCGATGGAGCCGATG   1080
                   *         *         *         *         *         *

 361   IleMetLeuProGluGlyLeuProGlyAlaGlyGlyTyrArgGluHisAspIleLeuIle    380
1081   ATCATGCTGCCGGAAGGCCTGCCGGGCGCCGGTGGCTATCGCGAGCACGACATCCTGATC   1140
                   *         *         *         *         *         *

 381   ValAsnGluAsnGlyAlaGluAsnIleThrLysPheProTyrGlyProGluLysAsnIle    400
1141   GTCAACGAGAACGGTGCCGAGAACATCACCAAGTTCCCCTACGGCCCGGAGAAAAACATC   1200
                   *         *         *         *         *         *

 401   IleArgLys *                                                     420
1201   ATCCGCAAATGA                                                   1260
                   *         *         *         *         *         *
```